# EUROPEAN PATENT SPECIFICATION

(11) **EP 3 820 526 B1**
(45) Date of publication and mention of the grant of the patent: **24.01.2024**
(21) Application number: 19734822.0
(22) Date of filing: 05.07.2019
(51) Int. Cl.: A61K 45/06, A61K 31/195, A61K 31/216, A61K 31/427, A61K 31/4439, A61P 1/16, A61P 11/00, A61P 27/00, A61P 9/00, A61P 17/00, A61P 13/12, A61K 31/155, A61K 31/415, A61K 31/44, A61K 31/519

(54) **COMBINATIONS OF PPAR AGONISTS AND P38 KINASE INHIBITORS FOR PREVENTING OR TREATING FIBROTIC DISEASES**
KOMBINATIONEN VON PPAR AGONISTEN UND P38 KINASE INHIBITOREN ZUR VORBEUGUNG ODER BEHANDLUNG VON FIBROTISCHEN ERKRANKUNGEN
COMBINAISONS D'AGONISTES DE PPAR ET D'INHIBITEURS DE LA KINASE P38 POUR LA PRÉVENTION OU LE TRAITEMENT DE MALADIES FIBROTIQUES

(30) Priority: 13.07.2018 EP 18183317
(43) Date of publication of application: 19.05.2021
(73) Proprietor: Kinarus AG, 4057 Basel (CH)
(72) Inventor: BAUSCH, Alexander, 4057 Basel (CH); WRIGHT, Matthew, 4057 Basel (CH)
(74) Representative: Latscha Schöllhorn Partner AG
(86) International application number: PCT/EP2019/068079
(87) International publication number: WO 2020/011661

(56) References cited:
- HEATHER F. LAKATOS ET AL: "The Role of PPARs in Lung Fibrosis", PPAR RESEARCH, vol. 2007, 1 January 2007 (2007-01-01), pages 1-10, XP055538465, US ISSN: 1687-4757, DOI: 10.1155/2007/71323
- H. TANIGUCHI ET AL: "Pirfenidone in idiopathic pulmonary fibrosis", EUROPEAN RESPIRATORY JOURNAL, vol. 35, no. 4, 8 December 2009 (2009-12-08), pages 821-829, XP055538463, ISSN: 0903-1936, DOI: 10.1183/09031936.00005209

## Description

### The field of the invention

The present invention relates to methods of preventing or treating fibrotic diseases or disorders.

### Background of the invention

Fibrotic disorders are often devastating diseases leading to loss of specific organ function and poor quality of life. In the United States, the prevalence of fibroproliferative diseases is increasing due to aging of the population and the increase in obesity and metabolic disorders. Approximately 45% of deaths in the U.S. have been associated with the presence of one or more fibroproliferative disorders.

Fibrosis involves an excess accumulation of extracellular matrix (primarily composed of collagen) and usually results in loss of function when normal tissue is replaced with acellular scar tissue. Fibrotic diseases often affect specific organs. Fibrotic diseases of the liver include liver cirrhosis, which can result from chronic hepatitis B or C infection, diabetes, obesity, autoimmune injury, and chronic exposure to toxins including alcohol. Fibrotic diseases of the kidney are a consequence of kidney injury and can contribute to renal failure. The presence of tubulointerstitial and glomerular fibrosis in the kidney reflects chronic, progressive renal disease and is considered the primary mechanism leading to end-stage renal disease. The presence of Type I and II diabetes mellitus commonly underlie the development of renal fibrosis. Chronic renal insufficiency is often a sign of renal fibrosis and can be caused by exposure to chemicals and nephrotoxic drugs. In addition, genetic causes and infection can cause renal fibrosis. For example, fibrosis and loss of function of heart tissue is caused by hypoxic and ischemic events that reduce blood flow and tissue oxygenation. Cardiac myocytes may die and be replaced by fibrotic tissue.

For example, fibrotic diseases of the lung include interstitial lung disease, characterized by pulmonary inflammation and fibrosis. Such diseases of the lung may have specific causes including underlying vascular disease, sarcoidosis, silicosis, or systemic scleroderma. In most cases, however, the cause is unknown. Idiopathic pulmonary fibrosis is a rare but devastating disorder with no known cause. IPF is detected by differential diagnosis where other causes, such as the ones noted, are excluded. Interstitial lung diseases (II,Ds) are associated with lung dysfunction due to fibrosis. Idiopathic Pulmonary Fibrosis (IPF) is a type of ILD that affects about 170,000 people in Europe and 130,000 people in the United States. IPF is a fatal disease with median survival of 3-5 years from diagnosis. Less than 30% survive beyond 5-years. Until recently, no effective treatment options other than lung transplantation had been shown to be effective. The most common cause of death is respiratory failure due to the progressive loss of lung function.

Recently, Nintedanib, a receptor tyrosine kinase inhibitor, was approved by the FDA for treatment of IPF. However, it is associated with significant systemic adverse events, including GI adverse reactions, liver enzyme elevation, decreased appetite, headache, weight loss, and hypertension. Pirfenidone was also approved by FDA in 2014 for the treatment of IPF. The mechanism of action of pirfenidone in the treatment of IPF has not been established. Pirfenidone must be administered at a high dose (801 mg) three times daily with meals. Pirfenidone also has significant side effects including nausea, rash, abdominal pain, upper respiratory tract infection, diarrhea, fatigue, headache, dyspepsia, dizziness, vomiting, anorexia, gastro-esophageal reflux disease, and sinusitis. More importantly, both nintedanib and pirfenidone are only partially effective, reducing the rate of decline in lung function by approximately 50%. Neither drug affects all-cause mortality or improves quality of life of patients with IPF. The role of PPARs in lung fibrosis is discussed in a review article written by Heather et al. (PPAR Research, Vol. 2007, 1 January 2007, pages 1-10). The authors conclude that the mechanisms by which PPAR ligands alter fibrosis are not well understood and that the relationship between the PPARs and fibrosis is likely to be complex.

Therefore, there remains a need for effective antifibrotic approaches to treat fibrotic diseases and, in particular, to improve the efficacy and tolerability of treatments for lung fibrotic diseases including IPF.

### Summary of the invention

It has now unexpectedly been found that a pharmaceutical combination comprising a PPAR agonist, such as pioglitazone and a p38 inhibitor, e.g. a compound of formula I or II as defined herein below, such as pamapimod, is useful for preventing or treating fibrotic diseases or disorders, in particular lung fibrotic diseases, such as IPF. In a standard model established in IPF research, it was surprisingly found that treatment with the pharmaceutical combination of the invention provides a greater effect to reduce fibrosis of the lung than treatment with a PPAR agonist or a p38 inhibitor alone and provides improved efficacy and tolerability when delivered orally. Moreover, the pharmaceutical combination was unexpectedly found to synergistically regulate the expression of multiple inflammatory genes of the interleukin/interleukin receptor, TNF/TNF receptor, and C-C and C-X-C motif chemokine gene families, indicating potentially potent anti-inflammatory effects, not exhibited by either agent alone.

Accordingly, in a first aspect, the present invention provides a pharmaceutical combination comprising:
(a) a PPAR agonist;
(b) a p38 kinase inhibitor; and optionally
(c) one or more pharmaceutically acceptable diluents, excipients or carriers for use in a method of preventing or treating fibrotic diseases or disorders in a subject, wherein the PPAR agonist is selected from the group consisting of pioglitazone, rosiglitazone, troglitazone and pharmaceutically acceptable salts thereof and, wherein the p38 kinase inhibitor is pamapimod or dilmapimod or pharmaceutically acceptable salts thereof.

In a further aspect, the present invention provides a kit for use in a method of preventing or treating fibrotic diseases or disorders in a subject, comprising a pharmaceutical combination comprising:
(a) a PPAR agonist;
(b) a p38 kinase inhibitor; and optionally
(c) one or more pharmaceutically acceptable diluents, excipients or carriers; and instructions for using the kit, wherein the PPAR agonist is selected from the group consisting of pioglitazone, rosiglitazone, troglitazone and pharmaceutically acceptable salts thereof and, wherein the p38 kinase inhibitor is pamapimod or dilmapimod or pharmaceutically acceptable salts thereof.

### Brief description of the figures

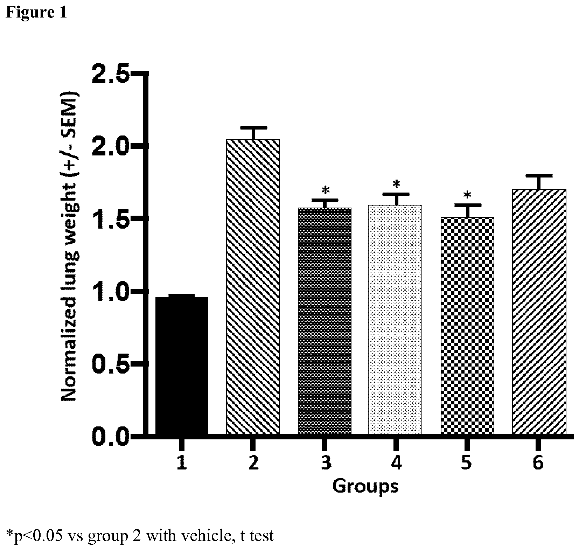

**Figure 1** shows lung weights normalized to individual animal body weights. Bleomycin instillation led to a significant increase in normalized lung weight when compared to non-bleomycin instilled sham controls. Substantially lower normalized lung weights were observed in groups treated with 25 mg/kg pioglitazone or 100 mg/kg pamapimod either alone or in combination (Groups 3, 4, and 5 vs. group 2, p<0.05, t test). Notably the combination reduced normalized lung weights to a greater extent than the positive control pirfenidone.

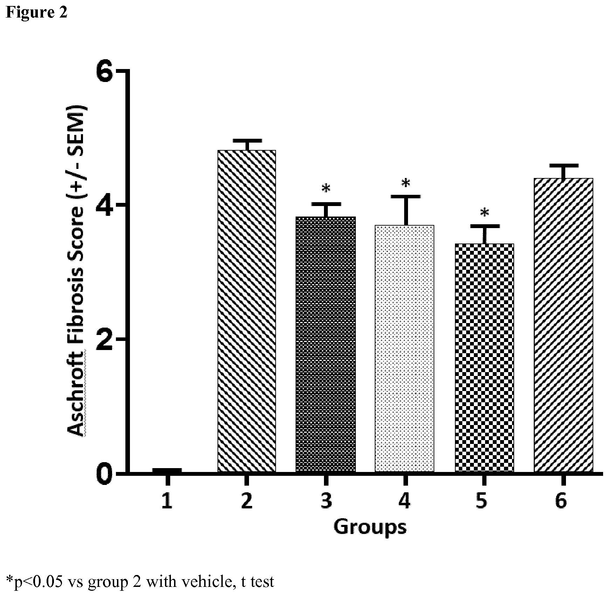

**Figure 2** shows the effect on fibrosis score in each treatment group. Induction with 1.5 U/kg bleomycin followed by 200 µL vehicle daily (Group 2) resulted in substantial fibrosis and the highest group mean fibrotic index (4.8). Substantially less fibrosis, as indicated by lower group mean fibrotic indices, was present in groups treated with 25 mg/kg pioglitazone or 100 mg/kg pamapimod either alone or in combination (Groups 3, 4, and 5 vs. group 2, p<0.05, t test). The lowest group mean fibrotic index was in Group 5, indicating that the combination of pioglitazone and pamapimod is more effective to reduce fibrosis score than either agent alone. Notably the combination reduced normalized fibrosis score to a greater extent than the positive control pirfenidone.

### Detailed description of the invention

For the purposes of interpreting this specification, the following definitions will apply and whenever appropriate, terms used in the singular will also include the plural and vice versa. It is to be understood that the terminology used herein is for the purpose of describing particular embodiments only and is not intended to be limiting.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any embodiment. The invention extends to any novel one, or any novel combination, of the features disclosed in this specification (including any accompanying claims, abstract and drawings), or to any novel one, or any novel combination, of the steps of any method or process so disclosed.

The terms "comprising", "having", and "including" are to be construed as open-ended terms (i.e., meaning "including, but not limited to") unless otherwise noted.

The term "pharmaceutically acceptable diluents, excipients or carriers" as used herein refers to diluents, excipients or carriers that are suitable for use with humans and/or animals without undue adverse side effects (such as toxicity, irritation, and allergic response) commensurate with a reasonable benefit/risk ratio. "Diluents" are agents which are added to the bulk volume of the active agent making up the solid composition. As a result, the size of the solid composition increases, which makes it easier to handle. Diluents are convenient when the dose of drug per solid composition is low and the solid composition would otherwise be too small. "Excipients" can be binders, lubricants, glidants, coating additives or combinations thereof. Thus, excipients are intended to serve multiple purposes. "Carriers" can be solvents, suspending agents or vehicles, for delivering the instant compounds to a subject.

The term "fibrotic diseases or disorders" is intended to refer to medical conditions of the body known in the art related to diseases or disorders caused by organs which develop excess fibrous connective tissue with impaired function. Accordingly, the term "fibrotic diseases or disorders" is meant to include, but is not limited to, diseases or disorders selected from the group consisting of lung fibrosis, liver fibrosis, kidney fibrosis, cardiac fibrosis, ocular fibrosis or cutaneous fibrosis.

The term "lung fibrosis" refers to a group of fibrotic diseases or disorders affecting the lung, such as idiopathic pulmonary fibrosis (IPF), familial interstitial pulmonary fibrosis, nonspecific interstitial pneumonia (NSIP), cryptogenic organizing pneumonia (COP), sarcoidosis, chronic obstructive pulmonary disease (COPD), and asbestosis. "Fibrotic diseases or disorders affecting the lung" or "fibrotic lung disease or disorder" are terms which are used interchangeably herein and refer to a respiratory disease in which scars are formed due to the accumulation of excess fibrous connective tissue in the lung tissues, mainly collagen, leading to progressive loss of lung function. The accumulation of excess fibrous connective tissue leads to thickening of alveolar walls, leading to reduced lung function. As a consequence, patients suffer from shortness of breath, are unable to exert themselves physically, and are at risk for pneumonia or other serious lung infections. In some patients, the specific cause of the disease can be diagnosed, but in others the probable cause cannot be determined, a condition called idiopathic pulmonary fibrosis (IPF). There are no treatments to reverse fibrosis or prevent further loss of lung function. Recently, Nintedanib, a receptor tyrosine kinase inhibitor, was approved by the FDA for treatment of IPF. However, it is associated with significant systemic adverse events, including GI adverse reactions, liver enzyme elevation, decreased appetite, headache, weight loss, and hypertension. Pirfenidone was also approved by FDA in 2014 for the treatment of IPF. Pirfenidone also has significant side effects including nausea, rash, abdominal pain, upper respiratory tract infection, diarrhea, fatigue, headache, dyspepsia, dizziness, vomiting, anorexia, gastro-esophageal reflux disease, and sinusitis. More importantly, both nintedanib and pirfenidone are only partially effective, reducing the rate of decline in lung function by approximately 50%. Neither drug affects all-cause mortality or improves quality of life of patients with IPF.

The term "idiopathic pulmonary fibrosis (IPF)" refers to a disease of the lung that occurs in middle-aged and elderly adults (median age at diagnosis 66 years, range 55-75 years). IPF is limited to the lungs, and is associated with a histopathological or radiological pattern typical of usual interstitial pneumonia. The cause of IPF is unknown, however a history of smoking, genetic factors, and environmental insults are thought to trigger the pathological changes that lead to fibrotic remodeling of lung tissue. After lung injury, epithelial cells release inflammatory mediators that initiate an anti-fibrinolytic coagulation cascade, which triggers platelet activation and blood clot formation. This is followed by entry of leukocytes (e.g., neutrophils, macrophages, and T cells). The recruited leukocytes secrete pro-fibrotic cytokines such as IL-1β, TNF-α, and TGF-β. In the subsequent phase, fibroblasts and myofibroblasts are derived from epithelial cells undergoing epithelial to mesenchymal transition, as well as fibrocytes from the bone marrow, and resident fibroblasts that proliferate and differentiate into myofibroblasts. These cells release collagen and other fibrotic components. IPF is a chronic, progressive, irreversible, and eventually lethal lung disease. Life expectancy after diagnosis ranges from 3-5 years.

The term "familial interstitial pulmonary fibrosis" refers to a disease similar to IPF, in which affects two or more related individuals. Familial interstitial pulmonary fibrosis has been suggested to be associated with changes in telomere length or gene mutations.

The term " nonspecific interstitial pneumonia (NSIP)" refers to a disease caused by reactions to certain medications, HIV, as well as other conditions. The disease is characterized mainly by inflammation of the cells of the interstitium. The fibrotic form is defined by thickening and scarring of lung tissue.

The term "cryptogenic organizing pneumonia (COP)" refers to a disease with alveolar inflammatory changes similar to regular pneumonia, but also with involvement of the bronchioles. Histologically, COP is characterised by mild patchy interstitial inflammation without fibrosis, and the presence of buds of granulation tissue made of mononuclear cells, foamy macrophages, and fibrous tissue (Masson bodies) in distal airspaces.

The term "sarcoidosis" refers to a disease involving abnormal collections of inflammatory cells into lumps called granulomas. When it affects the lungs there may be wheezing, coughing, shortness of breath, or chest pain.

The term chronic obstructive pulmonary disease (COPD) refers to a chronic inflammatory lung disease that causes obstructed airflow from the lungs. Symptoms include breathing difficulty, cough, mucus (sputum) production and wheezing.

The term "asbestosis" refers to a disease characterized by long term inflammation and scarring of the lungs due to asbestos exposure.

The term "pharmaceutically acceptable salt" of a compound means a salt that is pharmaceutically acceptable and that it possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxy-benzoyl)benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, e. g. an alkaline metal ion, an alkaline earth metal ion, or an aluminum ion; or coordinates with an organic base such as ethanolamine, diethanolamine, triethanolamine, tromethamine, N-methylglucamine, and the like. Preferred salts comprise acid addition salts formed with hydrochloric acid.

The terms "subject" and "patient" are used herein interchangeably and refer to mammals, in particular humans.

The term "about" as used herein refers to +/- 10 % of a given measurement.

In a first aspect, the present invention provides a pharmaceutical combination comprising:
(a) a PPAR agonist;
(b) a p38 kinase inhibitor; and optionally
(c) one or more pharmaceutically acceptable diluents, excipients or carriers for use in a method of preventing or treating fibrotic diseases or disorders in a subject, wherein the PPAR agonist is selected from the group consisting of pioglitazone, rosiglitazone, troglitazone and pharmaceutically acceptable salts thereof and, wherein the p38 kinase inhibitor is pamapimod or dilmapimod or pharmaceutically acceptable salts thereof.

### PPAR agonists

The term "PPAR agonist" as used herein refers to a drug that is activating peroxisome proliferator activated receptor (PPAR) such as PPAR gamma receptor, PPAR alpha receptor, PPAR delta receptor or combinations thereof and includes PPAR gamma agonists such as e.g. pioglitazone, troglitazone or rosiglitazone, PPAR alpha agonists such as e.g. fibrates such as bezafibrate, fenofibrate (fenofibric acid), clofibrate or gemfibrozil, PPAR dual agonists (PPAR alpha/gamma or PPAR alpha/delta agonists) such as e.g. aleglitazar, muraglitazar, tesaglitazar, ragaglitazar, saroglitazar, GFT505 or naveglitazar, PPAR delta agonists such as e.g. GW501516, PPAR pan agonists (PPAR alpha/delta/gamma agonists) or selective PPAR modulators such as e.g. INT131 and the pharmaceutically acceptable salts of these compounds. PPAR gamma agonists selected from the group consisting of pioglitazone, rosiglitazone, troglitazone and pharmaceutically acceptable salts thereof, preferably pioglitazone or pharmaceutically acceptable salts thereof are used in the pharmaceutical combinations of the present invention. Even more preferably, pioglitazone or a pharmaceutically acceptable salt thereof, in particular pioglitazone hydrochloride is used in the pharmaceutical combinations of the present invention.

Pioglitazone is described e.g. in US Patent No. 4,687,777 or in Dormandy JA, Charbonnel B, Eckland DJ, Erdmann E, Massi-Benedetti M, Moules IK, Skene AM, Tan MH, Lefèbvre PJ, Murray GD, Standl E, Wilcox RG, Wilhelmsen L, Betteridge J, Birkeland K, Golay A, Heine RJ, Korányi L, Laakso M, Mokán M, Norkus A, Pirags V, Podar T, Scheen A, Scherbaum W, Schernthaner G, Schmitz O, Skrha J, Smith U, Taton J; PROactive investigators. Lancet. 2005 Oct 8;366(9493): 1279-89, and is represented by the structural formula indicated below:

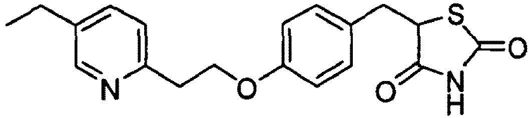

Troglitazone is described e.g. in Florez JC, Jablonski KA, Sun MW, Bayley N, Kahn SE, Shamoon H, Hamman RF, Knowler WC, Nathan DM, Altshuler D; Diabetes Prevention Program Research Group. J Clin Endocrinol Metab. 2007 Apr;92(4):1502-9 and is represented by the structural formula indicated below:

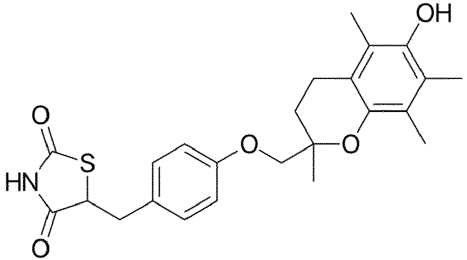

Rosiglitazone is described e.g. in Nissen SE, Wolski K. N Engl J Med. 2007 Jun 14;356(24):2457-71. Erratum in: N Engl J Med. 2007 Jul 5;357(1):100. Fenofibrate is described e.g. in Bonds DE, Craven TE, Buse J, Crouse JR, Cuddihy R, Elam M, Ginsberg HN, Kirchner K, Marcovina S, Mychaleckyj JC, O'Connor PJ, Sperl-Hillen JA. Diabetologia. 2012 Jun;55(6):1641-50 and is represented by the structural formula indicated below:

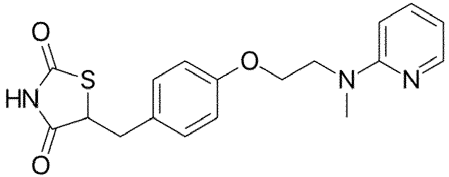

PPAR activation by the PPAR agonist is usually strong in the low nanomolar range to low micromolar range, e.g in a range of 0.1 nM to 100 µM. In some embodiments the PPAR activation is weak or partial, i.e. a PPAR agonist is used in the methods of the present invention which yields maximal activation of PPAR-receptor in a reporter assay system of 10% to 100% compared to a reference PPAR agonist which is known to causes a maximum PPAR activation.

### p38 kinase inhibitors

The term "p38 kinase inhibitor" or "p38 inhibitor" which are both used interchangeably herein refers to a drug that is inhibiting a p38 mitogen-activated protein (MAP) kinase, such as p38-alpha (MAPK14), p38-beta (MAPK11), p38-gamma (MAPK12 / ERK6), and/or p38-delta (MAPK13 / SAPK4). Examples of p38 inhibitors include pamapimod, acumapimod, losmapimod, dilmapimod, semapimod, AZD7624, ARRY-371797, LY2228820, R9111, PH-797804, BIRB 796, VX-702, VX-745, SB 239063, SB202190, SCIO 469, BMS 582949 and pharmaceutically acceptable salts thereof.

The p38 inhibitor for use in a pharmaceutical combination according to the invention is pamapimod or dilmapimod or pharmaceutically acceptable salts thereof in particular pamapimod or a pharmaceutically acceptable salt thereof.

In a particularly preferred embodiment, the p38 inhibitor is pamapimod, having the chemical name 6-(2,4-Difluorophenoxy)-2-[3-hydroxy-1-(2-hydroxyethyl)-propylamino]-8-methyl-8*H-*pyrido[2,3-*d*]pyrimidin-7-one and the chemical formula III or a pharmaceutically acceptable salt thereof.

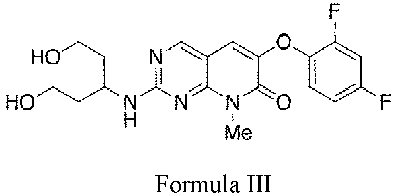

Pamapimod and its synthesis are described e.g. in WO2008/151992 and in WO2002/064594 and in e.g. Hill RJ, Dabbagh K, Phippard D, Li C, Suttmann RT, Welch M, Papp E, Song KW, Chang KC, Leaffer D, Kim Y-N, Roberts RT, Zabka TS, Aud D, Dal Porto J, Manning AM, Peng SL, Goldstein DM, and Wong BR; Pamapimod, a Novel p38 Mitogen-Activated Protein Kinase Inhibitor: Preclinical Analysis of Efficacy and Selectivity J Pharmacol Exp Ther. December 2008 327:610-619.

Dilmapimod is described in e.g. Christie JD, Vaslef S, Chang PK, May AK, Gunn SR, Yang S, Hardes K, Kahl L, Powley WM, Lipson DA, Bayliffe AI, Lazaar AL. A Randomized Dose-Escalation Study of the Safety and Anti-Inflammatory Activity of the p38 Mitogen-Activated Protein Kinase Inhibitor Dilmapimod in Severe Trauma Subjects at Risk for Acute Respiratory Distress Syndrome. Crit Care Med. 2015 Sep;43(9): 1859-69, and is represented by the structural formula indicated below:

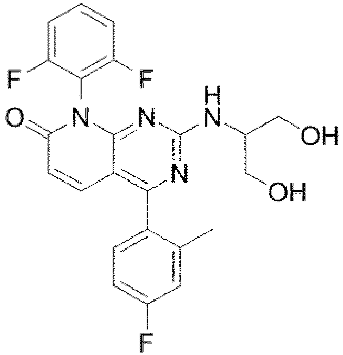

### Pharmaceutical combinations

As outlined above, in a first aspect, the present invention provides a pharmaceutical combination comprising:
(a) a PPAR agonist;
(b) a p38 kinase inhibitor; and optionally
(c) one or more pharmaceutically acceptable diluents, excipients or carriers for use in a method of preventing or treating fibrotic diseases or disorders in a subject, wherein the PPAR agonist is selected from the group consisting of pioglitazone, rosiglitazone, troglitazone and pharmaceutically acceptable salts thereof and, wherein the p38 kinase inhibitor is pamapimod or dilmapimod or pharmaceutically acceptable salts thereof.

Useful PPAR agonists are as defined above: A PPAR gamma agonist selected from the group consisting of pioglitazone, rosiglitazone and troglitazone or pharmaceutically acceptable salts thereof, in particular pioglitazone or a pharmaceutically acceptable salt thereof. In a particularly preferred embodiment, said PPAR agonist is pioglitazone hydrochloride.

Useful p38 kinase inhibitors are as defined above: Pamapimod or dilmapimod or pharmaceutically acceptable salts thereof, more particularly pamapimod or a pharmaceutically acceptable salt thereof.

A pharmaceutical combination according to the invention is for example a combined preparation or a pharmaceutical composition, for simultaneous, separate or sequential use. The term "combined preparation" as used herein defines especially a "kit of parts" in the sense that said PPAR agonist and said p38 inhibitor can be dosed independently, either in separate form e.g. as separate tablets or by use of different fixed combinations with distinguished amounts of the active ingredients. The ratio of the amount of PPAR agonist to the amount of p38 inhibitor to be administered in the combined preparation can be varied, e.g. in order to cope with the needs of a patient sub-population to be treated or the needs of a single patient, which needs can be different due to age, sex, body weight, etc. of a patient. The individual parts of the combined preparation (kit of parts) can be administered simultaneously or sequentially, i.e. chronologically staggered, e.g. at different time points and with equal or different time intervals for any part of the kit of parts.

The term "pharmaceutical composition" refers to a fixed-dose combination (FDC) that includes the PPAR agonist and the p38 inhibitor combined in a single dosage form, having a predetermined combination of respective dosages.

The pharmaceutical combination further may be used as add-on therapy. As used herein, "add-on" or "add-on therapy" means an assemblage of reagents for use in therapy, the subject receiving the therapy begins a first treatment regimen of one or more reagents prior to beginning a second treatment regimen of one or more different reagents in addition to the first treatment regimen, so that not all of the reagents used in the therapy are started at the same time. For example, adding p38 inhibitor therapy to a patient already receiving PPAR agonist therapy and vice versa.

In a preferred embodiment, the pharmaceutical combination according to the invention is a combined preparation.

In a further preferred embodiment, the pharmaceutical combination according to the invention is a pharmaceutical composition, i.e. a fixed-dose combination.

The amount of the PPAR agonist and the p38 inhibitor to be administered will vary depending upon factors such as the particular compound, disease condition and its severity, according to the particular circumstances surrounding the case, including, e.g., the specific PPAR agonist being administered, the route of administration, the condition being treated, the target area being treated, and the subject or host being treated.

In one embodiment, the invention provides a pharmaceutical combination comprising a PPAR agonist and a p38 inhibitor, wherein said PPAR agonist and said p38 inhibitor are present in a therapeutically effective amount.

In a preferred embodiment, the invention provides a pharmaceutical combination comprising a PPAR agonist and a p38 inhibitor, wherein said PPAR agonist and said p38 inhibitor produce an additive therapeutic effect i.e. wherein said PPAR agonist and said p38 inhibitor are present in an amount producing an additive therapeutic effect.

As used herein, the term "additive" means that the effect achieved with the pharmaceutical combinations of this invention is approximately the sum of the effects that result from using the agents, namely the PPAR agonist and the p38 inhibitor, as a monotherapy.

Advantageously, an additive effect provides for greater efficacy at the same doses, and may lead to longer duration of response to the therapy.

In a further preferred embodiment, the invention provides a pharmaceutical combination comprising a PPAR agonist and a p38 inhibitor, wherein said PPAR agonist and said p38 inhibitor produce a synergistic therapeutic effect, i.e. wherein said PPAR agonist and said p38 inhibitor are present in an amount producing a synergistic therapeutic effect.

In a further more preferred embodiment, the invention provides a pharmaceutical combination comprising a PPAR agonist and a p38 inhibitor, wherein said PPAR agonist and said p38 inhibitor produce a synergistic therapeutic effect in relation to the regulation of the expression of interleukin/interleukin receptor and/or TNF/TNF receptor genes, and/or C-C and C-X-C motif chemokine gene families, more particular wherein said PPAR agonist and said p38 inhibitor produce a synergistic therapeutic effect in relation to the regulation of the expression of one or more interleukin/interleukin receptor genes selected from the group consisting of interleukin 6, interleukin 11, interleukin 12B, and interleukin 18 receptor 1 and/or in relation to the regulation of the expression of TNF/TNF receptor genes selected from the group consisting of TNF receptor superfamily member 11b, TNF superfamily member 9, TNF receptor superfamily member 10b, and TNF superfamily member 18 and/or in relation to the regulation of the expression of the C-C and C-X-C motif chemokine genes selected from the group consisting of C-C motif chemokine ligand 7, C-C motif chemokine ligand 4, C-C motif chemokine ligand 3, C-C motif chemokine ligand 12, C-C motif chemokine ligand 2, C-C motif chemokine ligand 8, C-C motif chemokine ligand 24, C-X-C motif chemokine ligand 10, C-X-C motif chemokine ligand 5, C-X-C motif chemokine ligand 3, even more particular wherein said PPAR agonist and said p38 inhibitor produce a synergistic therapeutic effect in relation to the regulation of the expression of one or more interleukin/interleukin receptor genes selected from the group consisting of interleukin 6, interleukin 11, interleukin 12B, and interleukin 18 receptor 1 and/or in relation to the regulation of the expression of TNF/TNF receptor genes selected from the group consisting of TNF receptor superfamily member 11b, TNF superfamily member 9, TNF receptor superfamily member 10b, and TNF superfamily member 18.

As used herein, the term "synergistic" means that the effect achieved with the pharmaceutical combinations of this invention is greater than the sum of the effects that result from using the agents, namely the PPAR agonist and the p38 inhibitor, as a monotherapy. Advantageously, such synergy provides greater efficacy at the same doses and may lead to longer duration of response to the therapy.

In one embodiment, the invention provides a pharmaceutical combination comprising a p38 inhibitor and a PPAR agonist, wherein the amount of said PPAR agonist in the combination is from about 0.1 to about 45 mg, from about 0.1 to about 30 mg or from about 0.1 to about 15 mg. Where said PPAR agonist is in the form of a pharmaceutically acceptable salt, the amounts of PPAR agonist provided herein are calculated on the basis of the respective free base.

In a preferred embodiment, the invention provides a pharmaceutical combination comprising a p38 inhibitor and pioglitazone or a pharmaceutically acceptable salt thereof, wherein the amount of pioglitazone or a pharmaceutically acceptable salt thereof in the combination is below the dose typically needed for the treatment of diabetes with pioglitazone or a pharmaceutically acceptable salt thereof.

In one embodiment, the invention provides a pharmaceutical combination comprising a p38 inhibitor and a PPAR agonist, wherein the amount of said p38 inhibitor in the combination is from about 1 to about 500 mg or from about 1 to about 450 mg or from about 1 to about 400 mg or from about 1 to about 350 mg or from about 1 to about 300 mg or from about 1 to about 250 mg or from about 1 to about 200 mg or from about 1 to about 150 mg or from about 1 to about 125 mg or from about 1 to about 100 mg or from about 10 to about 125 mg or from about 10 to about 100 mg or from about 20 to about 100 mg or from about 30 to about 100 mg or from about 40 to about 100 mg or from about 50 to about 100 mg.

In a preferred embodiment, the pharmaceutical combination of the invention is a pharmaceutical composition (i.e. a fixed-dose combination, as outlined above). In one embodiment, the pharmaceutical combination of the invention is a pharmaceutical composition and includes other medicinal or pharmaceutical agents, e.g., one or more pharmaceutically acceptable diluents, excipients or carriers.

### Modes of Administration and Treatment

The terms "treatment"/"treating" as used herein includes: (1) delaying the appearance of clinical symptoms of the state, disorder or condition developing in an animal, particularly a mammal and especially a human, that may be afflicted with or predisposed to the state, disorder or condition but does not yet experience or display clinical or subclinical symptoms of the state, disorder or condition; (2) inhibiting the state, disorder or condition (e.g. arresting, reducing or delaying the progression of the disease, or a relapse thereof in case of maintenance treatment, of at least one clinical or subclinical symptom thereof); and/or (3) relieving the condition (i.e. causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

Preventive treatments comprise prophylactic treatments. In preventive applications, the pharmaceutical combination of the invention is administered to a subject suspected of having, or being at risk for developing fibrotic diseases or disorders. In therapeutic applications, the pharmaceutical combination of the invention is administered to a subject such as a patient already suffering from fibrotic diseases or disorders, in an amount sufficient to cure or at least partially arrest the symptoms of the disease. Amounts effective for this use will depend on the severity and course of the disease, previous therapy, the subject's health status and response to the drugs, and the judgment of the treating physician. In the case wherein the subject's condition does not improve, the pharmaceutical combination of the invention may be administered chronically, which is, for an extended period of time, including throughout the duration of the subject's life in order to ameliorate or otherwise control or limit the symptoms of the subject's disease or condition.

In the case wherein the subject's status does improve, the pharmaceutical combination of the invention may be administered continuously; alternatively, the dose of drugs being administered may be temporarily reduced or temporarily suspended for a certain length of time (i.e., a "drug holiday").

The pharmaceutical combination according to the invention is, preferably, suitable for oral, topical, injectable, ocular, local ocular (e.g., subconjunctival, intravitreal, retrobulbar or intracameral), systemic (i.e. enteral or parenteral) administration or suitable for administration by inhalation i.e. the combination is administered locally to the lung. More preferably the pharmaceutical combination according to the invention is suitable for oral, topical, injectable administration and/or administration by inhalation, most preferably suitable for oral administration to a subject and comprises a therapeutically effective amount of the active ingredient(s) and optionally one or more suitable pharmaceutically acceptable diluents, excipients or carriers.

If not indicated otherwise, a pharmaceutical combination according to the invention is prepared in a manner known per se, e.g. by means of conventional mixing, granulating, coating, dissolving or lyophilizing processes. In preparing a combination for an oral dosage form, any of the usual pharmaceutical media may be employed, carriers, diluents, granulating agents, lubricants, binders, disintegrating agents and the like. Because of their ease of administration, tablets and capsules represent the most advantageous oral dosage unit forms, in which case solid pharmaceutical carriers are obviously employed.

In a preferred embodiment, the pharmaceutical combination according to the invention is a combination for oral administration. As indicated above, said pharmaceutical combination for oral administration is preferably a pharmaceutical composition, i.e. a fixed-dose combination.

In one embodiment, the pharmaceutical combination according to the invention is a combination for topical administration. As indicated above, said pharmaceutical combination for topical administration is preferably a pharmaceutical composition, i.e. a fixed-dose combination.

In another preferred embodiment, the pharmaceutical combination according to the invention is a combination for injectable administration. As indicated above, said pharmaceutical combination for injectable administration is preferably a pharmaceutical composition, i.e. a fixed-dose combination.

In one embodiment, the pharmaceutical combination according to the invention is a combination for local ocular administration. As indicated above, said pharmaceutical combination for local ocular administration is preferably a pharmaceutical composition, i.e. a fixed-dose combination.

In another preferred embodiment, the pharmaceutical combination according to the invention is a combination for systemic, i.e. enteral or parenteral administration. As indicated above, said pharmaceutical combination for systemic administration is preferably a pharmaceutical composition, i.e. a fixed-dose combination.

In another preferred embodiment, the pharmaceutical combination according to the invention is a combination for administration by inhalation, for example as a nasal spray, or dry powder or aerosol inhalers. For delivery by inhalation, the active compounds are preferably in the form of microparticles. They may be prepared by a variety of techniques, including spray-drying, freeze-drying and micronisation. Aerosol generation can be carried out using, for example, pressure-driven jet atomizers or ultrasonic atomizers, preferably using propellant-driven metered aerosols or propellant-free administration of micronized active compounds from, for example, inhalation capsules or other "dry powder" delivery systems. Microparticles for delivery by inhalation may be formulated with excipients that aid delivery and release. For example, in a dry powder formulation, microparticles may be formulated with large carrier particles that aid flow from the DPI into the lung. Suitable carrier particles are known, and include lactose particles; they may have a mass median aerodynamic diameter of greater than 90 µm.

The active compounds may be dosed as described depending on the inhaler system used. In addition to the active compounds, the administration forms may additionally contain excipients, such as, for example, propellants (e.g. Frigen in the case of metered aerosols), surface-active substances, emulsifiers, stabilizers, preservatives, flavorings, fillers (e.g. lactose in the case of powder inhalers) or, if appropriate, further active compounds.

For the purposes of inhalation, a large number of systems are available with which aerosols of optimum particle size can be generated and administered, using an inhalation technique which is appropriate for the patient. In addition to the use of adaptors (spacers, expanders) and pear-shaped containers (e.g. Nebulator^{®}, Volumatic^{®}), and automatic devices emitting a puffer spray (Autohaler^{®}), for metered aerosols, in particular in the case of powder inhalers, a number of technical solutions are available (e.g. Diskhaler^{®}, Rotadisk^{®}, Turbohaler^{®} or the inhalers for example as described EP-A-0505321). Additionally, the combination of the invention may be delivered in multi-chamber devices thus allowing for separate storage and dosing of the PPAR agonist and the p38 inhibitor according to the invention.

In a more preferred embodiment the pharmaceutical combination according to the invention is administered orally, topically, by injection or by inhalation, even more preferably orally to the subject.

In a preferred embodiment the fibrotic diseases or disorders is lung fibrosis and the pharmaceutical combination according to the invention is a combination for oral administration, i.e. is administered orally. As indicated above, said pharmaceutical combination for oral administration is preferably a pharmaceutical composition, i.e. a fixed-dose combination.

In a further preferred embodiment the fibrotic diseases or disorders is lung fibrosis and the pharmaceutical combination according to the invention is a combination for administration by inhalation, i.e. is administered to the lung. As indicated above, said pharmaceutical combination for administration by inhalation is preferably a pharmaceutical composition, i.e. a fixed-dose combination.

In one embodiment the fibrotic diseases or disorders is liver fibrosis and the pharmaceutical combination according to the invention is a combination for oral administration, i.e. is administered orally. As indicated above, said pharmaceutical combination for oral administration is preferably a pharmaceutical composition, i.e. a fixed-dose combination.

In one embodiment the fibrotic diseases or disorders is kidney fibrosis and the pharmaceutical combination according to the invention is a combination for oral administration, i.e. is administered orally. As indicated above, said pharmaceutical combination for oral administration is preferably a pharmaceutical composition, i.e. a fixed-dose combination.

In one embodiment the fibrotic diseases or disorders is ocular fibrosis and the pharmaceutical combination according to the invention is a combination for oral, injection or topical administration, i.e. is administered orally, by injection or topically. As indicated above, said pharmaceutical combination for oral, injection or topical administration is preferably a pharmaceutical composition, i.e. a fixed-dose combination.

In one embodiment the fibrotic diseases or disorders is cutaneous fibrosis and the pharmaceutical combination according to the invention is a combination for oral or topical administration, i.e. is administered orally or topically. As indicated above, said pharmaceutical combination for oral or topical administration is preferably a pharmaceutical composition, i.e. a fixed-dose combination.

A pharmaceutical combination for oral or systemic i.e. enteral or parenteral administration is, for example, a unit dosage form, such as a tablet, a capsule or a suppository.

In one embodiment, the invention provides a pharmaceutical composition comprising a PPAR agonist, such as pioglitazone and a p38 inhhibitor, such as pamapimod and at least one pharmaceutically acceptable carrier, wherein the composition is a solution or a suspension for ocular administration (i.e. eye drops), or an ophthalmic ointment.

In one embodiment, the invention provides a pharmaceutical composition comprising a PPAR agonist, such as pioglitazone and a p38 inhhibitor, such as pamapimod and at least one pharmaceutically acceptable carrier, wherein the composition is a tablet or a capsule, preferably a tablet.

The unit content of active ingredients in an individual dose need not in itself constitute a therapeutically effective amount, since such an amount can be reached by the administration of a plurality of dosage units. A composition according to the invention may contain, e.g., from about 10% to about 100% of the therapeutically effective amount of the active ingredients.

Where the pharmaceutical combination according to the invention is a combined preparation, said PPAR agonist need not be administered in the same form as said p38 inhibitor. As an example, the PPAR agonist may be administered as a powder by inhalation, while the p38 inhibitor may be administered orally as a tablet or vice versa.

In some embodiments the pharmaceutical combination of the invention is administered to the subject in a dose that comprises a dose of a PPAR agonist which is below the dose needed for the treatment of diabetes using said PPAR agonist. In some embodiments the pharmaceutical combination of the invention is administered to the subject in a dose that comprises a dose of a PPAR agonist which is a factor of 3-9 fold lower than the top dose evaluated and tested for the treatment of diabetes, in particular a factor of 3-9 fold lower than the top dose evaluated and tested for the treatment of diabetes in human. The top dose evaluated and tested for the treatment of diabetes in human, e.g. for a PPAR gamma agonists such as pioglitazone hydrochloride, is usually in the range of about 15-45 mg/day. In some embodiments at the PPAR agonist dose used, the side effects seen in the treatment of diabetes using said PPAR agonist are reduced or not present.

In some embodiments the pharmaceutical combination of the invention is administered to the subject in a dose that comprises a dose of a PPAR agonist which is below the active dose for therapeutically relevant antidiabetic or anti-dyslipidemic effect of the PPAR agonist, in particular a dose that is below the active dose for antidiabetic or anti-dyslipidemic effect of the PPAR agonist in human.

A typical dosing regimen of pioglitazone or a pharmaceutically acceptable salt thereof in the treatment of diabetes includes 15 to 45 mg pioglitazone once-daily.

In some embodiments, the pharmaceutical combination of the invention is administered orally to a human in a dose comprising a dose of a PPAR agonist selected from the group consisting of pioglitazone, rosiglitazone, and troglitazone and pharmaceutically acceptable salts thereof, , yet more preferably pioglitazone or a pharmaceutically acceptable salt thereof, most preferably pioglitazone hydrochloride of 0.1-45 mg/day, preferably 0.1-10 mg/day, more preferably about 5 mg/day; and comprising a dose of a p38 inhibitor selected from the group consisting of pamapimodand dilmapimod or a pharmaceutically acceptable salt thereof, more preferably pamapimod or a pharmaceutically acceptable salt thereof of 1-500 mg/day, preferably 10-250 mg/day, more preferably 25-150 mg/day, most preferably about 75 mg/day.

### Dosing regimen

An exemplary treatment regime entails administration once daily, twice daily, or thrice daily every second day, preferably once daily and/or twice daily . The combination of the invention is usually administered on multiple occasions. Intervals between single dosages can be, for example, less than a day, daily, or every second day. The combination of the invention may be given as a continous uninterrupted treatment. The combination of the invention may also be given in a regime in which the subject receives cycles of treatment interrupted by a drug holiday or period of non-treatment. Thus, the combination of the invention may be administered according to the selected intervals above for a continuous period of one week or a part thereof, for two weeks, for three weeks, for four weeks, for five weeks or for six weeks and then stopped for a period of one week, or a part thereof, for two weeks, for three weeks, for four weeks, for five weeks, or for six weeks. The combination of the treament interval and the non-treatment interval is called a cycle. The cycle may be repeated one or more times. Two or more different cycles may be used in combination for repeating the treatment one or more times. Intervals can also be irregular and guided either by worseining or improvement in the condition of the patient indicated by appearance or remission of symptoms or objective evidence of disease appearance or remission. In such case, therapy may be started and suspended as needed, and only restarted when symptoms or objective measures indicate the return of disease. In a preferred embodiment, the pharmaceutical combination according to the invention is administered once daily.

### Kits/Articles of Manufacture

In one aspect, the present invention also provides a kit for use in a method of preventing or treating fibrotic diseases or disorders in a subject, comprising a pharmaceutical combination disclosed herein, and instructions for using the kit. Preferred PPAR agonists and preferred p38 kinase inhibitors comprised by said pharmaceutical combination are as described above.

In some embodiments, kits include a carrier, package, or container that is compartmentalized to receive one or more containers such as vials, tubes, and the like, each of the container(s) including one of the separate elements to be used in a method described herein. Suitable containers include, for example, bottles, vials, syringes, and test tubes. In other embodiments, the containers are formed from a variety of materials such as glass or plastic.

The articles of manufacture provided herein generally will comprise one or more pharmaceutical combination disclosed herein and packaging materials. Examples of pharmaceutical packaging materials include, but are not limited to, blister packs, bottles, tubes, inhalers, pumps, bags, vials, containers, syringes, and any packaging material suitable for a selected composition and intended mode of administration and treatment.

### Preventing or treating fibrotic diseases or disorders

In one aspect, the present invention provides a pharmaceutical combination described herein, i.e. a pharmaceutical combination comprising:
(a) a PPAR agonist;
(b) a p38 kinase inhibitor; and optionally
(c) one or more pharmaceutically acceptable diluents, excipients or carriers for use in a method of preventing or treating fibrotic diseases or disorders in a subject, wherein the PPAR agonist is selected from the group consisting of pioglitazone, rosiglitazone, troglitazone and pharmaceutically acceptable salts thereof and, wherein the p38 kinase inhibitor is pamapimod or dilmapimod or pharmaceutically acceptable salts thereof.

Also provided is the use of a pharmaceutical combination described herein for the manufacture of a medicament for preventing or treating fibrotic diseases or disorders in a subj ect.

Also provided is the use of a pharmaceutical combination described herein for preventing or treating fibrotic diseases or disorders in a subject.

Also provided is a method of preventing or treating fibrotic diseases or disorders in a subject, comprising administering to said subject a therapeutically effective amount of a pharmaceutical combination as described herein.

In a preferred embodiment, the present invention provides a pharmaceutical combination described herein for use in a method of preventing or treating fibrotic diseases or disorders in a subject, wherein said fibrotic disease or disorder is selected from the group consisting of lung fibrosis, liver fibrosis, kidney fibrosis, cardiac fibrosis, ocular fibrosis or cutaneous fibrosis.

In a more preferred embodiment, the present invention provides a pharmaceutical combination described herein for use in a method of preventing or treating lung fibrosis.

In an even more preferred embodiment, the present invention provides a pharmaceutical combination described herein for use in a method of preventing or treating lung fibrosis, wherein the lung fibrosis is selected from the group consisting of idiopathic pulmonary fibrosis (IPF), familial interstitial pulmonary fibrosis, nonspecific interstitial pneumonia (NSIP), cryptogenic organizing pneumonia (COP), sarcoidosis, and asbestosis.

In a further even more preferred embodiment, the present invention provides a pharmaceutical combination described herein for use in a method of preventing or treating lung fibrosis, wherein the lung fibrosis is selected from the group consisting of idiopathic pulmonary fibrosis (IPF), familial interstitial pulmonary fibrosis, nonspecific interstitial pneumonia (NSIP), cryptogenic organizing pneumonia (COP), sarcoidosis, chronic obstructive pulmonary disease (COPD), and asbestosis.

In a particular preferred embodiment, the present invention provides a pharmaceutical combination described herein for use in a method of preventing or treating lung fibrosis, wherein the lung fibrosis is selected from the group consisting of idiopathic pulmonary fibrosis (IPF), familial interstitial pulmonary fibrosis and asbestosis.

In a further particular preferred embodiment, the present invention provides a pharmaceutical combination described herein for use in a method of preventing or treating lung fibrosis, wherein the lung fibrosis is selected from the group consisting of idiopathic pulmonary fibrosis (IPF), familial interstitial pulmonary fibrosis, chronic obstructive pulmonary disease (COPD), and asbestosis.

In a more particular preferred embodiment, the present invention provides a pharmaceutical combination described herein for use in a method of preventing or treating idiopathic pulmonary fibrosis (IPF).

In a further preferred embodiment, the present invention provides a pharmaceutical combination comprising
(a) a PPAR gamma agonist;
(b) a p38 kinase inhibitor, wherein said p38 kinase inhibitor is pamapimod or a pharmaceutically acceptable salt thereof; and optionally
(c) one or more pharmaceutically acceptable diluents, excipients or carriers for use in a method of preventing or treating fibrotic diseases or disorders in a subject; wherein said PPAR gamma agonist is selected from the group consisting of pioglitazone, rosiglitazone and troglitazone or pharmaceutically acceptable salts thereof.

In a particularly preferred embodiment, the present invention provides a pharmaceutical combination comprising:
(a) a PPAR agonist, wherein said PPAR agonist is pioglitazone or a pharmaceutically acceptable salt thereof, preferably pioglitazone hydrochloride;
(b) a p38 kinase inhibitor, wherein said p38 kinase inhibitor is pamapimod or a pharmaceutically acceptable salt thereof; and optionally
(c) one or more pharmaceutically acceptable diluents, excipients or carriers for use in a method of preventing or treating fibrotic diseases or disorders in a subject.

In a further particularly preferred embodiment, the present invention provides a pharmaceutical combination comprising:
(a) a PPAR agonist, wherein said PPAR agonist is pioglitazone or a pharmaceutically acceptable salt thereof, preferably pioglitazone hydrochloride;
(b) a p38 kinase inhibitor, wherein said p38 kinase inhibitor is pamapimod or a pharmaceutically acceptable salt thereof; and optionally
(c) one or more pharmaceutically acceptable diluents, excipients or carriers for use in a method of preventing or treating fibrotic diseases or disorders in a subject, wherein said fibrotic diseases or disorders is idiopathic pulmonary fibrosis (IPF).

### Examples

The present Examples are intended to illustrate the present invention without restricting it.

### Example 1: Combination treatment with pamapimod and pioglitazone protects against bleomycin-induced lung fibrosis

### 1. SUMMARY

Bleomycin is widely used to induce pulmonary fibrosis in mice in order to study potential therapies. The efficacy of pioglitazone and pamapimod, dosed individually, and in combination, in comparison to the approved drug pirfenidone, were tested in a 21-day model of bleomycin-induced pulmonary fibrosis in mice. Both once daily pioglitazone and once daily pamapimod, dosed individually and in combination, significantly reduced normalized lung weights and fibrosis scores compared to vehicle-treated controls. The combination of pamapimod/pioglitazone group showed greater reductions in these disease paramenters than the groups receiving the single agents. All treatment groups showed greater efficacy than the positive control group receiving twice daily pirfenidone. These results show that the combination of pioglitazone and pamapimod is effective to reduce bleomycin-induced lung fibrosis and is a candidate therapeutic regimen for the treatment of lung fibrosis.

### 2. MATERIALS AND METHODS

### 2.1. Study Animals

Male C57BL/6 mice, six to seven weeks of age, were used for this study. Animals were weighed one day prior to study initiation and 60 animals were randomized into 6 groups (N=10/group) such that mean body weights were similar for the different groups. Food and water were provided ad libitum, with a 12-hour light/dark cycle.

### 2.2. Study Design

Treatment in groups 2-6 was initiated one day prior to bleomycin administration and continued though to the study end on day 21. On day -1, animals were dosed via oral gavage with vehicle control (0.5% methylcellulose; group 1 and group 2), pioglitazone at 25 mg/kg/dose (group 3), pamapimod at 100 mg/kg/dose (group 4), pioglitazone at 25 mg/kg/dose and pamapimod at 100 mg/kg/dose (group 5), pirfenidone at 100mg/kg/dose (group 6) and continued for 21 days. Pioglitazone and pamapimod were dosed once daily (QD) and pirfenidone was dosed twice daily (BID). On day 0, all mice in groups 2-6 received a single instillation of bleomycin to induce pulmonary fibrosis. Animals in group 1 (dosed with vehicle from days -1 to 21) were not instilled with bleomycin, but instead received a single dose of saline, and were considered sham negative control mice. Final drug treatments were administered 2-4 hours prior to study termination on day 21.

Body weights, absolute lung weights, and lung weights normalized to body weights were measured for all mice on day 21 after bleomycin instillation, and were compared to bleomycin-instilled vehicle treated control mice. The post-caval lobes from the right lung were collected and snap frozen. The remaining lungs were fixed in 10 % NBF and underwent histopathology analysis and evaluated for eseverity of fibrosis using a modified Ashcroft score.

### 2.3. Compounds and Vehicle Formulation

Vehicle (0.5% methylcellulose) and compounds were prepared weekly and stored at room temperature in the dark. All mice were dosed starting on day -1 until study termination on day 21 with a volume of 200 µl of vehicle or test compounds QD by oral gavage or 100 µl of pirfenidone BID by oral gavage. For groups receiving both pirfenidone and test compounds, pirfenidone was dosed 100 µl BID and test compounds were delivered in a total volume of 100 µl QD.

### 2.4. Harvest Procedures

This study was terminated on day 21 for all groups approximately 2 to 4 hours after final compound or vehicle control treatment. All mice were anesthetized with isoflurane inhalant anesthesia and sacrificed by cervical dislocation.

The lungs from each animal were harvested and weighed. The post-caval lobe from the right lung was separated by tying a suture so that there was no leakage from the rest of the lung. The post caval lobe was weighed and snap frozen for further analysis. The remaining lungs from each animal were inflated with 10% neutral buffered formalin (NBF) and then fixed in 10% NBF for histology.

### 2.5. Fibrosis scoring

The lung samples were processed and embedded with all lobes from each mouse in one paraffin block. Coronal sections through the four major lobes were stained with Masson's Trichrome. For each animal, consecutive lung fields were examined in a raster pattern using a 20X objective lens and a 10X ocular lens (200X). A modified Ashcroft score (Hubner et al., 2008) was recorded for each field. The fibrotic index was calculated as the sum of the modified Ashcroft field scores divided by the number of fields examined. Results of the histopathology microscopic evaluation were entered directly into Excel 2016 spreadsheets.

Endpoint data on day 21 is shown as the average Ashcroft score per experimental group, with standard error of the mean (SEM). Statistical differences (defined as p<0.05) between groups were analyzed using t-tests in Prism version 7.0 software (GraphPad, La Jolla, CA). Groups treated with test compounds were compared to bleomycin-instilled vehicle control treated mice.

### 3. RESULTS

### 3.1. Absolute and Normalized Lung Weights

As shown by Figure 1, bleomycin instillation led to a significant increase in normalized lung weight when compared to non-bleomycin instilled sham controls. Substantially lower normalized lung weights were observed in groups treated with 25 mg/kg pioglitazone or 100 mg/kg pamapimod either alone or in combination (Groups 3, 4, and 5 vs. group 2, p<0.05, t test). Notably the combination reduced normalized lung weights to a greater extent than the positive control pirfenidone (Group 6).

### 3.2. Fibrosis score

As shown by Figure 2, induction with 1.5 U/kg bleomycin followed by 200 µL vehicle daily (Group 2) resulted in substantial fibrosis and the highest group mean fibrotic index (4.8).

Substantially less fibrosis, as indicated by lower group mean fibrotic indices, was present in groups treated with 25 mg/kg pioglitazone or 100 mg/kg pamapimod either alone or in combination (Groups 3, 4, and 5 vs. group 2, p<0.05, t test). The lowest group mean fibrotic index was in Group 5, indicating that the combination of pioglitazone and pamapimod is more effective to reduce fibrosis score than either agent alone. Notably the combination reduced normalized fibrosis score to a greater extent than the positive control pirfenidone (Group 6).

### Example 2: Combination treatment with pamapimod and pioglitazone shows synergy on Interleukin and TNF pathway gene expression changes in mouse lung tissue

### 1. SUMMARY

Fibrosis is a pathological process characterized by the replacement of normal tissue by mesenchymal cells and extracellular matrix. The sequence of events leading to fibrosis of the lung involves inflammation and disruption of normal tissue architecture. Several cytokines participate in local injury and inflammation. These include interleukin-1 (IL-1), interleukin-8 (IL-8), Interleukin-6 as well as tumor necrosis factor-alpha (TNF-alpha)] and related molecules. We performed whole genome expression analysis on lung tissue from the bleomycin fibrosis study presented in Example 1. Surprisingly, the results revealed that pioglitazone and pamapimod have a synergistic effect to significantly regulate the expression of multiple interleukin/interleukin receptor as well as TNF/TNF receptor genes. These data suggest that the combination of the two drugs may provide better therapeutic efficacy in the treatment of lung fibrosis than either drug alone.

### 2. MATERIALS AND METHODS

Global gene expression changes were determined by Illumina Next Generation RNA sequencing in lung samples from the animal study described in Example 1. RNA was extracted from the post caval lobe that had been snap frozen at the termination of the study. For this analysis, 8 samples from each of treatment groups 1-6 (total 48 samples) were analysed. Briefly, total RNA was extracted for next generation sequencing using standard methods, then Illumina TruSeq RNA libraries including poly(A) enrichment were prepared. Sequencing was performed on an Illumina NextSeq 500, v2, high output, 1x75 bp reads with 30 Mio packages. Demultiplexing and trimming of Illumina adaptor residuals was performed on the raw data. Mapping of data reads was made using the reference mouse genome mm10.

For bioinformatics analysis, the average expression level and standard deviations were determined for each gene for the 8 replicates per treatment group. Pairwise comparisons between groups were then performed to identify differentially expressed genes. Absolute p-values and adjusted p-values, to correct for multiplicity of testing, were calculated for each pairwise analysis. Data output consisted of the top 1000 differentially expressed genes. A gene was considered significantly up or downregulated if the difference in a between-group comparison yielded an adjusted p value < 0.10.

### 3. RESULTS

Table 1 below shows the significantly regulated genes (adjusted p value < 0.10) for the three treatment groups in comparison to the the bleomycin alone disease group. Neither pioglitazone or pamapimod single treatments significantly regulated expression of members of the interleukin/interleukin receptor, TNF/TNF receptor, or C-C and C-X-C motif chemokine families. Surprisingly, combined treatment with pioglitazone/pamapimod significantly repressed a very large number of inflammatory cytokines belonging to these four families. Many of these have been implicated in the pathogenesis of IPF. These data strongly support synergy of the combination, indicating that the combination has potentially potent anti-inflammatory effects, not exhibited by either agent alone.

**Table 1: Synergistic effect of the pamapimod/pioglitazone combination on cytokines and chemokines of the interleukin, TNF, C-C and C-X-C motif families in the mouse bleomycin model**

| **RNASeq whole genome lung expression data from Mouse bleomycin study 2** | | | | | |
|---|---|---|---|---|---|
| | | | | | |

| **Interleukins and receptors** | | **adjusted significance (p) value** | | | |
|---|---|---|---|---|---|
| **Gene** | **Gene ID** | **Pioglitazone** | **Pamapimod** | **Combination** | **Direction** |
| Interleukin 6 | IL6 | NS | NS | **0.000161** | downregulated |
| Interleukin 12B | IL12b | NS | **0.023** | **0.0142** | downregulated |
| Interleukin 36 Gamma | IL1f9 | NS | NS | **0.033** | upregulated |
| Interleukin 11 | IL11 | NS | NS | **0.054** | downregulated |
| Interleukin 18 Receptor 1 | IL18r1 | NS | NS | **0.076** | downregulated |
| Interleukin 1 Receptor Type 2 | IL1r2 | NS | NS | **0.084** | upregulated |
| | NS = adjusted p value > 0.10 | | | | |
| | | | | | |

| **TNFs and receptors** | | **adjusted significance (p) value** | | | |
|---|---|---|---|---|---|
| **Gene** | **Gene ID** | **Pioglitazone** | **Pamapimod** | **Combination** | **Direction** |
| TNF Receptor Superfamily Member 11b | Tnfrsfllb | NS | NS | **0.0084** | downregulated |
| TNF Superfamily Member 9 | Tnfsf9 | NS | NS | **0.028** | downregulated |
| TNF Receptor Superfamily Member 10b | TnfrsflOb | NS | NS | **0.055** | downregulated |
| TNF Superfamily Member 18 | Tnfsf18 | NS | NS | **0.073** | downregulated |
| TNF Superfamily Member 15 | Tnfsf15 | NS | **0.0079** | NS | upregulated |
| | NS = adjusted p value > 0.10 | | | | |
| | | | | | |

| **Proinflammatory chemokines** | | **adjusted significance (p) value** | | | |
|---|---|---|---|---|---|
| **Gene** | **Gene ID** | **Pioglitazone** | **Pamapimod** | **Combination** | **Direction** |
| C-C Motif Chemokine Ligand 7 | Ccl7 | NS | NS | **0.0014** | downregulated |
| C-C Motif Chemokine Ligand 4 | Ccl4 | NS | 0.042 | **0.0032** | downregulated |
| C-C Motif Chemokine Ligand 3 | Ccl3 | NS | NS | **0.0048** | downregulated |
| C-C Motif Chemokine Ligand 12 | Ccl12 | NS | NS | **0.021** | downregulated |
| C-C Motif Chemokine Ligand 2 | Ccl2 | NS | NS | **0.023** | downregulated |
| C-C Motif Chemokine Ligand 8 | Ccl8 | NS | NS | **0.08** | downregulated |
| C-C Motif Chemokine Ligand 24 | Ccl24 | NS | **0.032** | NS | downregulated |
| C-X-C Motif Chemokine Ligand 10 | Cxcl10 | NS | NS | **0.048** | downregulated |
| C-X-C Motif Chemokine Ligand 5 | Cxcl5 | NS | NS | **0.052** | downregulated |
| C-X-C Motif Chemokine Ligand 3 | Cxcl3 | NS | NS | **0.094** | downregulated |
| | NS = adjusted p value > 0.10 | | | | |

## Claims

1. A pharmaceutical combination comprising:
(a) a PPAR agonist;
(b) a p38 kinase inhibitor; and optionally
(c) one or more pharmaceutically acceptable diluents, excipients or carriers for use in a method of preventing or treating fibrotic diseases or disorders in a subject, wherein the PPAR agonist is selected from the group consisting of pioglitazone, rosiglitazone, troglitazone and pharmaceutically acceptable salts thereof and, wherein the p38 kinase inhibitor is pamapimod or dilmapimod or pharmaceutically acceptable salts thereof.

2. The pharmaceutical combination for use according to claim 1, wherein said p38 kinase inhibitor is pamapimod (6-(2,4-Difluorophenoxy)-2-[3-hydroxy-1-(2-hydroxyethyl)-propylamino]-8-methyl-8*H*-pyrido[2,3-*d*]pyrimidin-7-one, Formula III) or a pharmaceutically acceptable salt thereof.

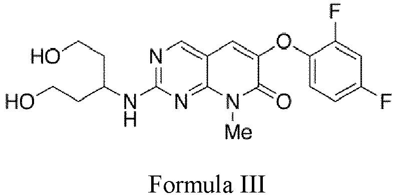

3. The pharmaceutical combination for use according to any one of claims 1-2, wherein said PPAR agonist is pioglitazone or a pharmaceutically acceptable salt thereof.

4. The pharmaceutical combination for use according to any one of claims 1-3, wherein said combination is administered orally to the subject.

5. The pharmaceutical combination for use according to any one of claims 1-4, wherein said fibrotic disease or disorder is selected from the group consisting of lung fibrosis, liver fibrosis, kidney fibrosis, cardiac fibrosis, ocular fibrosis or cutaneous fibrosis.

6. The pharmaceutical combination for use according to any one of claims 1-5, wherein said fibrotic disease or disorder is lung fibrosis.

7. The pharmaceutical combination for use according to claim 6, wherein said lung fibrosis is selected from the group consisting of idiopathic pulmonary fibrosis (IPF), familial interstitial pulmonary fibrosis, nonspecific interstitial pneumonia (NSIP), cryptogenic organizing pneumonia (COP), sarcoidosis, chronic obstructive pulmonary disease (COPD), and asbestosis.

8. A kit for use in a method of preventing or treating fibrotic diseases or disorders in a subject, comprising a pharmaceutical combination comprising:
(a) a PPAR agonist;
(b) a p38 kinase inhibitor; and optionally
(c) one or more pharmaceutically acceptable diluents, excipients or carriers; and instructions for using the kit, wherein the PPAR agonist is selected from the group consisting of pioglitazone, rosiglitazone, troglitazone and pharmaceutically acceptable salts thereof and, wherein the p38 kinase inhibitor is pamapimod or dilmapimod or pharmaceutically acceptable salts thereof.

## Patentansprüche

1. Pharmazeutische Kombination, umfassend:
(a) einen PPAR-Agonisten;
(b) einen p38-Kinaseinhibitor; und optional
(c) einen oder mehrere pharmazeutisch verträgliche Verdünnungsmittel, Hilfsstoffe oder Träger zur Verwendung in einem Verfahren zum Vorbeugen oder Behandeln fibrotischer Krankheiten oder Störungen bei einem Subjekt, wobei der PPAR-Agonist ausgewählt ist aus der Gruppe bestehend aus Pioglitazon, Rosiglitazon, Troglitazon und pharmazeutisch verträglichen Salzen davon und wobei es sich bei dem p38-Kinaseinhibitor um Pamapimod oder Dilmapimod oder pharmazeutisch verträgliche Salze davon handelt.

2. Pharmazeutische Kombination zur Verwendung nach Anspruch 1, wobei der p38-Kinaseinhibitor Pamapimod (6-(2,4-Difluorphenoxy)-2-[3-hydroxy-1-(2-hydroxyethyl)-propylamino]-8-methyl-8*H*-pyrido[2,3-*d*]pyrimidin-7-on, Formel III) oder ein pharmazeutisch verträgliches Salz davon ist.

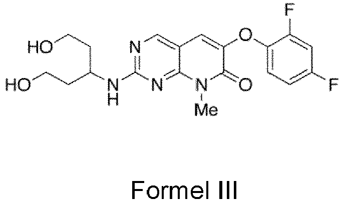

3. Pharmazeutische Kombination zur Verwendung nach einem der Ansprüche 1 bis 2, wobei der PPAR-Agonist Pioglitazon oder ein pharmazeutisch verträgliches Salz davon ist.

4. Pharmazeutische Kombination zur Verwendung nach einem der Ansprüche 1 bis 3, wobei die Kombination dem Subjekt oral verabreicht wird.

5. Pharmazeutische Kombination zur Verwendung nach einem der Ansprüche 1 bis 4, wobei die fibrotische Krankheit oder Störung aus der Gruppe ausgewählt ist bestehend aus Lungenfibrose, Leberfibrose, Nierenfibrose, Herzfibrose, Augenfibrose oder Hautfibrose.

6. Pharmazeutische Kombination zur Verwendung nach einem der Ansprüche 1 bis 5, wobei die fibrotische Krankheit oder Störung eine Lungenfibrose ist.

7. Pharmazeutische Kombination zur Verwendung nach Anspruch 6, wobei die Lungenfibrose ausgewählt ist aus der Gruppe bestehend aus idiopathischer Lungenfibrose (IPF), familiärer interstitieller Lungenfibrose, unspezifischer interstitieller Pneumonie (NSIP), kryptogener organisierender Pneumonie (COP), Sarkoidose, chronisch obstruktiver Lungenerkrankung (COPD) und Asbestose.

8. Kit zur Verwendung in einem Verfahren zum Vorbeugen oder Behandeln fibrotischer Krankheiten oder Störungen bei einem Subjekt, umfassend eine pharmazeutische Kombination, umfassend:
(a) einen PPAR-Agonisten;
(b) einen p38-Kinaseinhibitor; und optional
(c) einen oder mehrere pharmazeutisch verträgliche Verdünnungsmittel, Hilfsstoffe oder Träger;
und Anweisungen zur Verwendung des Kits, wobei der PPAR-Agonist ausgewählt ist aus der Gruppe bestehend aus Pioglitazon, Rosiglitazon, Troglitazon und pharmazeutisch verträglichen Salzen davon und wobei es sich bei dem p38-Kinaseinhibitor um Pamapimod oder Dilmapimod oder pharmazeutisch verträgliche Salze davon handelt.

## Revendications

1. Combinaison pharmaceutique comprenant :
(a) un agoniste de PPAR ;
(b) un inhibiteur de kinase p38 ; et éventuellement
(c) un ou plusieurs diluants, excipients ou vecteurs pharmaceutiquement acceptables pour utilisation dans un procédé de prévention ou de traitement de maladies ou troubles fibrotiques chez un sujet, dans laquelle l'agoniste de PPAR est choisi dans le groupe constitué de pioglitazone, rosiglitazone, troglitazone et sels pharmaceutiquement acceptables de celles-ci et, dans laquelle l'inhibiteur de kinase p38 est pamapimod ou dilmapimod ou des sels pharmaceutiquement acceptables de ceux-ci.

2. Combinaison pharmaceutique pour utilisation selon la revendication 1, dans laquelle ledit inhibiteur de kinase p38 est pamapimod (6-(2,4-difluorophénoxy)-2-[3-hydroxy-1-(2-hydroxyéthyl)-propylamino]-8-méthyl-8*H-*pyrido[2,3-*d*]pyrimidin-7-one, Formule III) ou un sel pharmaceutiquement acceptable de celui-ci.

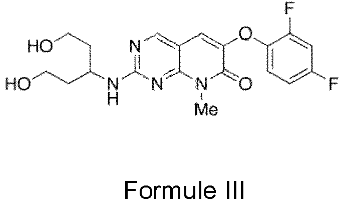

3. Combinaison pharmaceutique pour utilisation selon l'une quelconque des revendications 1 à 2, dans laquelle ledit agoniste de PPAR est la pioglitazone ou un sel pharmaceutiquement acceptable de celle-ci.

4. Combinaison pharmaceutique pour utilisation selon l'une quelconque des revendications 1 à 3, dans laquelle ladite combinaison est administrée par voie orale au sujet.

5. Combinaison pharmaceutique pour utilisation selon l'une quelconque des revendications 1 à 4, dans laquelle ladite maladie ou ledit trouble fibrotique est choisi dans le groupe constitué de fibrose pulmonaire, fibrose hépatique, fibrose rénale, fibrose cardiaque, fibrose oculaire ou fibrose cutanée.

6. Combinaison pharmaceutique pour utilisation selon l'une quelconque des revendications 1 à 5, dans laquelle ladite maladie ou ledit trouble fibrotique est une fibrose pulmonaire.

7. Combinaison pharmaceutique pour utilisation selon la revendication 6, dans laquelle ladite fibrose pulmonaire est choisie dans le groupe constitué de fibrose pulmonaire idiopathique (IPF), fibrose pulmonaire interstitielle familiale, pneumonie interstitielle non spécifique (NSIP), pneumonie organisée cryptogénique (COP), sarcoïdose, broncho-pneumopathie chronique obstructive (BPCO) et asbestose.

8. Trousse pour utilisation dans un procédé de prévention ou de traitement de maladies ou troubles fibrotiques chez un sujet, comprenant une combinaison pharmaceutique comprenant :
(a) un agoniste de PPAR ;
(b) un inhibiteur de kinase p38 ; et éventuellement
(c) un ou plusieurs diluants, excipients ou vecteurs pharmaceutiquement acceptables ;
et des instructions d'utilisation de la trousse, dans laquelle l'agoniste de PPAR est choisi dans le groupe constitué de pioglitazone, rosiglitazone, troglitazone ou sels pharmaceutiquement acceptables de celles-ci et, dans laquelle l'inhibiteur de kinase p38 est pamapimod ou dilmapimod ou des sels pharmaceutiquement acceptables de ceux-ci.
